(19) Europäisches Patentamt
European Patent Office
Office européen des brevets

(11) Veröffentlichungsnummer: **0 008 433**
**A1**

# (12) EUROPÄISCHE PATENTANMELDUNG

(21) Anmeldenummer: 79102951.5

(22) Anmeldetag: 14.08.79

(51) Int. Cl.³: C 07 D 263/10
C 07 C 143/78, A 61 K 31/42
A 61 K 31/235

(30) Priorität: 17.08.78 DE 2836085

(43) Veröffentlichungstag der Anmeldung: 05.03.80 Patentblatt 80/5

(84) Benannte Vertragsstaaten: AT BE CH DE FR GB IT NL

(71) Anmelder: HOECHST AKTIENGESELLSCHAFT
Zentrale Patentabteilung Postfach 80 03 20
D-6230 Frankfurt/Main 80(DE)

(72) Erfinder: Mania, Dieter, Dr.
Berliner Ring 5
D-6233 Kelkheim, (Taunus)(DE)

(72) Erfinder: Bormann, Dieter, Dr.
Johann-Strauss-Strasse 20
D-6233 Kelkheim, (Taunus)(DE)

(72) Erfinder: Merkel, Wulf, Dr.
Hattersheimer Strasse 14
D-6233 Kelkheim, (Taunus)(DE)

(72) Erfinder: Muschaweck, Roman, Dr.
Heimchenweg 39
D-6230 Frankfurt am Main 80(DE)

(72) Erfinder: Hropot, Max, Dr.
Friedrich-Stolz-Strasse 13
D-6093 Flörsheim am Main(DE)

(54) **Sulfamoylbenzolderivate, Verfahren zu ihrer Herstellung, pharmazeutische Präparate auf Basis dieser Verbindungen sowie deren Herstellung.**

(57) Halogensulfamoylbenzole der allgemeinen Formel

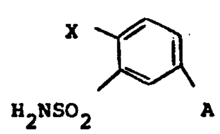

in der X für ein Halogen und A für einen der Reste

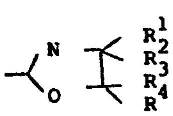

oder

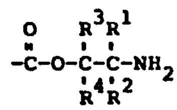

a b

steht, wobei die Reste R¹ bis R⁴ gleich oder verschieden sind und Wasserstoff oder einen Alkylrest mit 1 - 4 C-Atomen bedeuten, einer der Substituenten R¹ bis R⁴ eine Carboxy-, Hydroxymethyl- oder eine Alkyloxycarbonyl-Gruppe mit höchstens 5 C-Atomen bedeuten oder ein oder zwei der Substituenten R¹ bis R⁴ iso-Propyl, iso-Butyl, tert.-Butyl, Phenyl oder Cycloalkyl mit 5 - 6 C-Atomen bedeuten, während die übrigen für Wasserstoff oder niederes Alkyl stehen.

EP 0 008 433 A1

Croydon Printing Company Ltd.

0008433

HOECHST AKTIENGESELLSCHAFT HOE 78/F 170 Dr.LI/Rp

**Sulfamoylbenzolderivate, Verfahren zu ihrer Herstellung, pharmazeutische Präparate auf Basis dieser Verbindungen sowie deren Herstellung**

Gegenstand der Anmeldung sind Halogensulfamoylbenzole der allgemeinen Formel

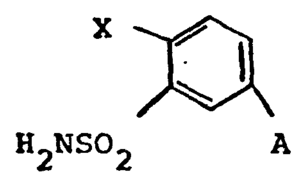

I a bzw. I b

in der X für ein Halogen und A für einen der Reste

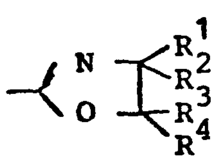

oder

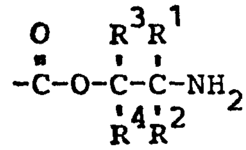

a b

steht, wobei die Reste $R^1$ bis $R^4$ gleich oder verschieden sind und Wasserstoff oder einen Alkylrest mit 1 - 4 C-Atomen bedeuten, einer der Substituenten $R^1$ bis $R^4$ eine Carboxy-, Hydroxymethyl- oder eine Alkyloxycarbonyl-Gruppe mit höchstens 5 C-Atomen bedeuten oder ein oder zwei der Substituenten $R^1$ bis $R^4$ iso-Propyl, iso-Butyl,

tert.-Butyl, Phenyl oder Cycloalkyl mit 5 - 6 C-Atomen bedeuten, während die übrigen für Wasserstoff oder niederes Alkyl stehen, sowie deren Salze mit physiologisch verträglichen Säuren.

Gegenstand der Erfindung ist weiterhin ein Verfahren zur Herstellung der Verbindungen der Formel I, das dadurch gekennzeichnet ist, daß man

a) Sulfamoylverbindungen der Formel II

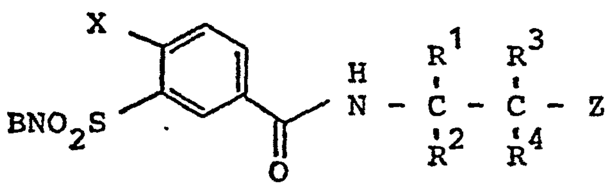

II

in der die Reste $R^1$ bis $R^4$ und X die angegebene Bedeutung haben und B für 2 Wasserstoffatome oder eine Schutzgruppe der Formel

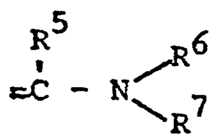

steht, in der die Reste $R^5$ bis $R^7$ gleiche oder verschiedene Alkylgruppen mit 1 - 4 C-Atomen bedeuten, wobei $R^5$ auch für Wasserstoff stehen kann und Z eine leaving group darstellt, cyclisiert oder

b) Sulfamoylverbindungen der Formel III

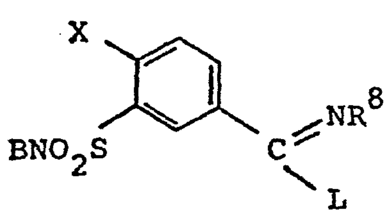

III

in der X und B die angegebenen Bedeutungen besitzen, $R^8$ Wasserstoff oder einen Alkylrest mit 1 - 4 C-Atomen bedeutet und L für Halogen, Alkoxy oder Dialkylamino, steht, worin die Alkyl- bzw. Alkoxyreste 1 - 4 C-Atome enthalten, in Form ihrer Salze mit Aminoalkoholen der allgemeinen Formel IV

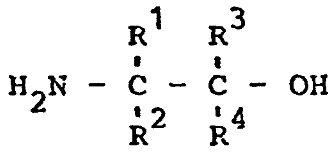

IV

in der die Reste $R^1$ bis $R^4$ die angegebene Bedeutung besitzen, umsetzt oder

c) Sulfamoylverbindungen der allgemeinen Formel V

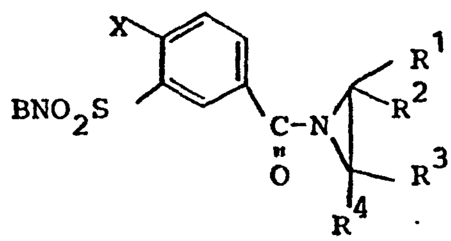

V

in der die Reste $R^1$ bis $R^4$, X und B die angegebene Bedeutung haben, durch Erwärmen oder Zusatz eines Katalysators zum Oxazolinderivat der allgemeinen Formel I a isomerisiert oder

d) ein Nitril der Formel VI

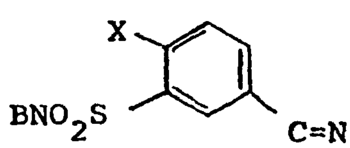

VI

in der X und B die angegebene Bedeutung besitzen, mit Epoxiden der allgemeinen Formel VII

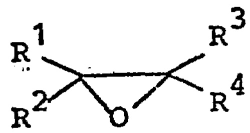

VII

worin $R^1$ bis $R^4$ die angegebene Bedeutung besitzen, jedoch nicht für Phenyl stehen, in Gegenwart starker Säuren umsetzt oder

e) ein Allylamid der Formel VIII

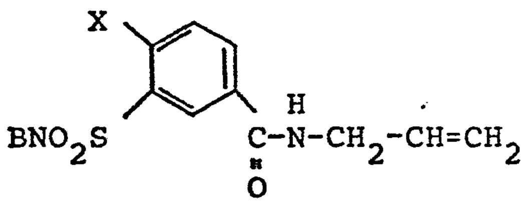

VIII

in der X und B die angegebene Bedeutung haben, mit Säuren cyclisiert,

und in den nach a) bis e) erhaltenen Verbindungen eine gegebenenfalls vorhandene Schutzgruppe durch alkalische Hydrolyse abspaltet und gegebenenfalls die nach a) bis e) erhaltenen Oxazolinderivate der Formel I a in Gegenwart von wäßrigen Säuren in der Wärme zu Verbindungen der Formel I b umlagert und/oder die Verbindungen der Formel I a bzw. I b mit physiologisch verträglichen organischen oder anorganischen Säuren in die Säure-Additionssalze und/oder die anfallenden Salze in die entsprechenden Basen überführt.

In den bei der Variante a eingesetzten Amiden der Formel II ist der Rest X vorzugsweise ein Chlor oder Bromatom. Als Reste $R^5$ bis $R^7$ in der Schutzgruppe B kommen beispielsweise Methyl-, Äthyl- oder Isopropylgruppen in Betracht, besonders bevorzugt sind Schutzgruppen B, in denen $R^5$ einen Wasserstoff- oder Methylrest und $R^6$ und $R^7$ Methylgruppen darstellen.

Als leaving group Z kommen insbesondere Halogenatome wie beispielsweise Brom oder Chlor, die Hydroxygruppe, tri-Alkylammonium, die Mesylat- oder Tosylatgruppen in Betracht.

Die ß-Hydroxyamide der allgemeinen Formel II in der Z für OH steht, werden auf verschiedenen Wegen hergestellt, beispielsweise durch Umsetzung von Verbindungen der Formel IX

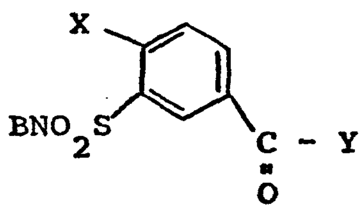

IX

in der X und B die angegebene Bedeutung besitzt und Y für eine leicht durch Amine substituierbare Gruppe, wie beispielsweise Halogen, insbesondere Chlor oder Brom, eine niedrig Alkoxy- bzw. Acyloxygruppe steht, mit Aminoalkoholen der allgemeinen Formel X

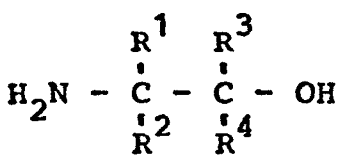

X

wobei die Reste $R^1$ bis $R^4$ die angegebene Bedeutung haben, jedoch nicht für Carboxyl oder Hydroxymethyl stehen, unter den Bedingungen der Schotten-Baumann-Reaktion umsetzt.

Die für die Umsetzung erforderlichen Verbindungen IX und X sind literaturbekannt oder lassen sich analog bekannter Vorschriften leicht herstellen.

Weitere Methoden zur Herstellung von Verbindungen der Formel II mit Z = OH sind zusammenfassend in Chem. Rev. 44, 447 (1949) beschrieben.

Die erfindungsgemäße Cyclisierung zu Oxazolinderivaten der allgemeinen Formel I a gemäß Verfahrensweise a) erfolgt unter verschiedenen Bedingungen. Beispielsweise lassen sich ß-Hydroxyamide der allgemeinen Formel II mit Z = OH durch Erhitzen auf 190 bis 250°, vorzugsweise bei 190 bis 220°, in die Oxazolinderivate der allgemeinen Formel Ia überführen, fernen kann durch Kochen im organischen Lösungsmittel das Wasser azeotrop entfernt und so der Ringschluß zum Oxazolin gefördert werden. Als Lösungsmittel eignen sich die bekannten Mittel zur azeotropen Destillation, wie beispielsweise Toluol, Xylol und die chlorierten Kohlenwasserstoffe wie z.B. Dichloräthylen.

Weiter lassen sich die Verbindungen der allgemeinen Formel II mit Z = OH mit wasserentziehenden Mitteln wie beispielsweise $H_2SO_4$, $P_2O_5$, $POCl_3$ bei 60 bis 160°, vorzugsweise 80 bis 100°, cyclisieren. Die Cyclisierung kann auch in einer tertiären Base, beispielsweise in Pyridin bei -10 bis +30°, vorzugsweise bei 0°, analog J. Am. Chem. Soc. 75, 5896 (1953) mit Tosyl- oder Mesylchlorid mit hohen Ausbeuten durchgeführt werden.

Eine weitere Cyclisierungsmethode ist die Umsetzung eines ß-Hydroxyamides der Formel II mit überschüssigem Thionylchlorid bei 0 bis 40°, vorzugsweise bei 15 bis 25°. Es werden hierbei die salzsauren Salze der Verbindungen der Formel I a gebildet.

Die Aufarbeitung des Reaktionsgemisches erfolgt in für den Fachmann geläufiger Weise, so wird beispielsweise das Thionylchlorid destillativ entfernt und anschließend das salzsaure Oxazolinderivat der Formel I a durch Behandeln mit kalter Lauge, vorzugsweise NaOH oder KOH bei einem pH oberhalb 12,5 gelöst und anschließend durch pH-Einstellung auf den Bereich von 12,5 bis 9 die freie Base der allgemeinen Formel I a gefällt.

Wurde von einer Verbindung der Formel II ausgegangen in der die $SO_2NH_2$-Gruppe durch die Schutzgruppe B blockiert ist, so wird letztere zunächst im alkalischen Milieu bei 40 bis 100°, vorzugsweise 50 bis 60°, hydrolysiert und anschließend das Oxazolinderivat der Formel I a gefällt.

Die ß-Halogenamide der Formel II, in der B, X und $R^1$ bis $R^4$ die angegebene Bedeutung haben, jedoch zweckmäßig nicht für Alkoxycarbonyl, Carboxy oder Hydroxymethyl stehen, und Z = Cl oder Br ist, lassen sich nach bekannten Verfahren - Chem. Rev. 44, 447 (1949), oder Band 71, 483 (1971) - herstellen und mit hochprozentiger Lauge insbesondere 10 bis 40 %iger NaOH oder KOH bei -10 bis 40° zu den Oxazolinderivaten der Formel I a cyclisieren.

Steht in der allgemeinen Formel II Z für eine tri-niedrig-Alkylammoniumgruppe, so kommen insbesondere die Alkylreste mit 1 bis 4 C-Atomen in Betracht wie beispielsweise die Trimethylammonium-, Triäthylammonium- oder Tributylammoniumgruppe. Diese Verbindungen können auf verschiedenen Wegen hergestellt werden, beispielsweise durch Umsetzung der Säurechloride der Formel IX mit den Aminen der Formel XI

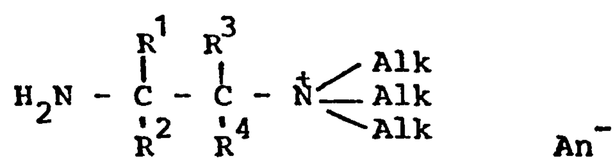
XI

in der die Reste $R^1$ bis $R^4$ die angegebene Bedeutung haben, $N(Alk)_3$ für tri-niedrig Alkylgruppe und $An^-$ für ein organisches oder anorganisches Anion steht, wie beispielsweise ein p-Toluolsulfonat, Chlorid oder Bromid. Die Cyclisierung dieser Verbindungen der Formel II erfolgt wie vorstehend für die ß-Halogenamide beschrieben.

Steht in der allgemeinen Formel II Z für eine Tosylat- oder Mesylatgruppe, so sind die Verbindungen auf verschiedenen Wegen zugänglich, beispielsweise durch Umsetzung der oben erwähnten ß-Hydroxyamide (Z = OH in der Formel II) mit p-Toluolsulfonsäurehalogenid, wie beispielsweise p-Toluolsulfonsäurechlorid oder -bromid sowie der entsprechenden Mesylverbindungen in Gegenwart von Basen. Als Basen kommen organische oder anorganische Basen in Betracht. Die Umsetzung dieser Verbindungen der Formel II erfolgt bevorzugt in einem Lösungsmittel, beispielsweise in Pyridin, das gleichzeitig als Base und als Lösungsmittel dient. Dabei ist es in vielen Fällen vorteilhaft, die Tosylate oder Mesylate nicht in Substanz zu isolieren, sondern sie direkt in Gegenwart des als Base wirkenden Lösungsmittels zu den Oxazolinderivaten der Formel I a zu cyclisieren.

Für die Umsetzung nach Verfahrensweise b) werden Verbindungen der Formel III mit Aminoalkoholen der Formel IV umgesetzt. Als Verbindungen der Formel III kann man Iminoäthersalze verwenden, d.h. hier bedeutet L eine niedermolekulare Alkoxygruppe, die zweckmäßig unverzweigt ist. Die Umsetzung kann mit oder ohne Lösungsmittel durchgeführt werden.

Als Lösungsmittel verwendet man organische Lösungsmittel wie beispielsweise Acetonitril, halogenierte Kohlenwasserstoffe, wie Methylenchlorid oder Chloroform. Besonders bevorzugt ist jedoch die Umsetzung der Komponenten ohne Lösungsmittel, wobei vorteilhaft die beiden Verbindungen miteinander verschmolzen werden. Die Isolierung der Oxazolinderivate erfolgt wie bei a) beschrieben.

Die als Ausgangsstoffe verwendeten Iminoäther der Formel III lassen sich nach bekannten Methoden (Chem. Rev. 71, 483 (1971) oder Houben-Weyl Band VIII, Seite 697 und Band XI/2 Seite 38 bis 66 (4. Auflage)) synthetisieren.

Die zur Umsetzung erforderlichen Amidiniumsalze der Formel III, worin L eine Dialkylaminogruppe bedeutet, lassen sich nach bekannten Methoden (Chem. Rev. 71, 483 (1971) synthetisieren.

Es kommt prinzipiell die gleiche Arbeitsweise in Betracht, wie bei der Umsetzung der Iminoäther beschrieben. Es werden die Komponenten vorzugsweise ohne Lösungsmittel bei 50 - 150°, insbesondere bei 80 bis 120° zu den Oxazolinderivaten der Formel Ia umgesetzt.

Die Imidchloridsalze der allgemeinen Formel III, in der X, B und $R^8$ die angegebene Bedeutung haben und L für ein Halogen, insbesondere Chlor steht, lassen sich in bekannter Weise (Houben-Weyl Band XI/2, Seite 38 bis 66) herstellen und mit überschüssigem Aminoalkohol der Formel IV zu den Oxazolinderivaten umsetzen. Man arbeitet in gleicher Weise wie bei der Umsetzung mit Iminoäther beschrieben.

Die Isolierung der Oxazolin-Derivate erfolgt wie oben beschrieben.

Die Ausgangsverbindungen der Formel X für die Verfahrensweise c) sind literaturbekannt oder können analog zu literaturbekannten Verfahren, beispielsweise aus den Säurechloriden der allgemeinen Formel IX in der X und B die oben angegebene Bedeutung haben, mit Aziridinen der allgemeinen Formel XII

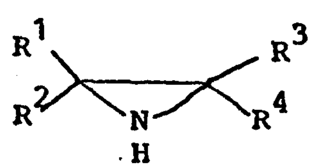

XII

in der $R^1$ bis $R^4$ die angegebene Bedeutung haben, jedoch zweckmäßig nicht für Alkoxycarbonyl, Carboxy oder Hydroxymethyl stehen, in einem die Reaktion nicht behindernden organischen Lösungsmittel, beispielsweise Aceton oder einem chlorierten Kohlenwasserstoff wie Chloroform, in Gegenwart einer tertiären Base z.B. Triäthylamin oder Pyridin hergestellt werden.

Die Isomerisierung der Verbindungen der allgemeinen Formel V zu Verbindungen der allgemeinen Formel Ia, in der X, B und $R^1$ bis $R^4$ die angegebene Bedeutung haben, wobei $R^1$ bis $R^4$ jedoch zweckmäßig nicht für eine Alkoxy-, Carboxy- oder Hydroxyäthylgruppe steht, wird in einem organischen Lösungsmittel beispielsweise Aceton, vorteilhaft durch Zusatz eines Katalysators begünstigt. Als Katalysatoren kommen vor allem Alkaliverbindungen mit stark nucleophilen Anionen, insbesondere NaJ, KSCN, $NaN_3$ oder Tributylamin in Betracht. Die Menge des zugesetzten Katalysators ist nicht kritisch, größere Mengen verkürzen jedoch die Reaktionszeit. Nach Zusatz des Katalysators wird 0,5 bis 3 Stunden unter Rückfluß gekocht und das entstandene Oxazolin wie oben beschrieben isoliert.

Nach der Verfahrensvariante d), der Umsetzung der literaturbekannten Nitrile der allgemeinen Formel VI mit den ebenfalls bekannte Epoxiden der Formel VII kommen starke Säuren, vor allem die technisch leicht zugänglichen Säuren wie konz. Schwefelsäure oder konz. Phosphorsäure in Betracht. Die Umsetzung wird vorzugsweise in der Kälte durchgeführt, etwa bei Temperaturen von -20 bis +10°. Zur Aufarbeitung wird mit Wasser verdünnt, anschließend durch Zusatz von KOH oder NaOH alkalisch gestellt und die Endprodukte wie beschrieben isoliert.

Nach der Verfahrensweise e) werden die literaturbekannten Allylamide der allgemeinen Formel VIIImit Säuren bei 50 bis 170°, vorzugsweise bei 80 bis 100°, zum Oxazolinderivat der allgemeinen Formel I a cyclisiert. In diesem Falle stehen in den Endprodukten der Formel I a $R^1$, $R^2$, $R^3$ für Wasserstoff und $R^4$ für Methyl. Als Säuren eignen sich beispielsweise die bei d) genannten anorganischen Säuren.

Im Anschluß an die Umsetzungen gemäß a) bis e) wird eine gegebenenfalls noch vorhandene Schutzgruppe B durch alkalische Hydrolyse abgespalten.

Die Salze der Oxazolinderivate der allgemeinen Formel I a lassen sich in einem Lösungsmittel, beispielsweise in Wasser bei 60 bis 100° in die Salze der allgemeinen Formel I b umlagern. Vorteilhaft setzt man verdünnte Säuren zu, man kann auch in Gegenwart von wassermischbaren organischen Lösungsmitteln arbeiten. Die freien Aminoester der allgemeinen Formel I b lassen sich durch Zusatz von Basen isolieren.

Die Verbindungen der allgemeinen Formel I a oder Ib lassen sich durch einen äquimolaren Zusatz einer physiologisch verträglichen Säure beispielsweise HCl, $H_2SO_4$, Maleinsäure, Bernsteinsäure oder Zitronensäure in die entsprechenden Salze überführen. Aus den Salzen lassen sich durch Zusatz von Basen, insbesondere NaOH oder KOH die basischen Verbindungen der allgemeinen Formel I a bzw. I b wiedergewinnen.

Die erfindungsgemäßen Verbindungen der Formel Ia und I b, sowie deren physiologisch verträglichen Salze sind wirksame Diuretika und Saluretika. Sie eigenen sich zur Behandlung von Ödemkrankheiten, wie kardiale, renale oder hepatisch bedingte Ödeme und anderer auf Störungen des Wasser- und Elektrolyt-Haushalts zurückzuführende Erscheinungen. Sie werden in Dosierungen von 0,5 bis 100 mg in Kapseln, Dragees, Tabletten oder Lösungen die ggf. pharmazeutisch übliche Trägerstoffe und/oder Stabilisatoren enthalten, oral oder parenteral verabfolgt. Die tägliche Dosierung liegt zwischen 50 und 1000 mg pro Patient.

Über die in den Beispielen beschriebenen Verbindungen lassen sich nach dem erfindungsgemäßen Verfahren die folgenden Substanzen herstellen.

4-Chlor-3-sulfamoyl— (4-methyl-oxazolin-2-yl)benzol
4-Chlor-3-sulfamoyl— (4-Propyl-oxazolin-2-yl)benzol
4-Chlor-3-sulfamoyl— (4-butyl —oxazolin-2-yl)benzol
4-Chlor-3-sulfamoyl— (5-äthyl-oxazolin-2-yl)benzol
4-Chlor-3-sulfamoyl— (5-propyl-oxazolin-2-yl)benzol
4-Chlor-3-sulfamoyl— (5-butyl-oxazolin-2-yl)benzol
4-Chlor-3-sulfamoyl— (cis-4-methyl-5-methyl-oxazolin-2-yl) benzol
4-Chlor-3-sulfamoyl— (trans-4-methyl-5-methyl-oxazolin-2-yl) benzol
4-Chlor-3-sulfamoyl— (4-phenyl-oxazolin-2-yl)benzol
4-Chlor-3-sulfamoyl— (4-cyclohexyl-oxazolin-2-yl)benzol
4-Chlor-3-sulfamoyl— (4-cyclopentyl-oxazolin-2-yl)benzol
4-Chlor-3-sulfamoyl— (5-cyclohexyl-oxazolin-2-yl)benzol
4-Chlor-3-sulfamoyl— (5-cyclopentyl-oxazolin-2-yl)benzol
4-Brom-3-sulfamoyl— (oxazolin-2-yl)benzol
4-Brom-3-sulfamoyl— (4-methyl-oxazolin-2-yl)benzol
4-Chlor-3-sulfamoyl— (4-hydroxymethyl-oxazolin-2-yl)benzol
4-Chlor-3-sulfamoyl-(4,4-dihydroxymethyl-oxazolin-2-yl)benzol
4-Chlor-3-sulfamoyl— (4-carbmethoxy-oxazolin-2-yl)benzol
4-Chlor-3-sulfamoyl— (4-carboxy-oxazolin-2-yl)benzol
4-Chlor-3-sulfamoyl— (4-carbäthoxy-5-methyl -oxazolin-2-yl)-benzol
4-Chlor-3-sulfamoyl— (4-carboxy-5-methyl oxazolin-2-yl)-benzol
4-Chlor-3-sulfamoyl— (4-hydroxymethyl-5-methyl -oxazolin-2-yl)-benzol
4-Chlor-3-sulfamoyl— (4-carbäthoxy-5-phenyl -oxazolin-2-yl)-benzol
4-Chlor-3-sulfamoyl— (4-carboxy-5-phenyl -oxazolin-2-yl)-benzol
4-Chlor-3-sulfamoyl— (4-hydroxymethyl-5-phenyl,oxazolin-2-yl)-benzol
4-Chlor-3-sulfamoyl— (4-methyl-5-phenyl -oxazolin-2-yl)-benzol

Beispiel 1

Herstellung der Verbindungen der Formel II:

a) 4-Chlor-3-sulfamoylbenzoesäure-(1-hydroxy-2-methyl-propyl-2-)-amid

25 g (~0,1 Mol) 4-Chlor-3-sulfamoylbenzoesäuremethyl-ester werden in 100 ml 2-Amino-2 -methylpropanol-1 2 Stunden bei 90° erwärmt. Nach Abdestillieren des überschüssigen Aminoalkohols im Hochvakuum wird der Rückstand in der Kälte mit ~ 150 ml Wasser verdünnt und mit 2 n HCl auf pH 1 - 2 angesäuert. Nach Stehen über Nacht im Eisschrank wird der Niederschlag abgesaugt, mit Wasser gewaschen, getrocknet und aus Methanol/Wasser umkristallisiert. Schmp. 186 - 187°C

b) 4-Chlor-3-sulfamoylbenzoesäure-(2-hydroxy-propyl)-amid

analog Beispiel b, mit 1-Amino-propanol-2 Kristalle vom Schmp. 166 - 168°

c) 4-Chlor-3-sulfamoylbenzoesäure(1-hydroxy-butyl-2-)amid

analog Beispiel b, mit 2-Amino-butanol-1 Kristalle vom Schmp. 201 - 203°

d) 4-Brom-3-sulfamoylbenzoesäure(1-hydroxy-butyl-2-)amid

analog Beispiel b, mit 4-Brom-3-sulfamoylbenzoesäure-methylester und 2-Amino-butanol-1 Kristalle vom Schmp. 215 - 217°

e) 4-Chlor-3-dimethylaminomethylenaminosulfonyl-benzoesäure-(2-hydroxy-2 -phenyl-äthyl)-amid

29 g (0,1 Mol) 4-Chlor-3-dimethylaminomethylenamino-sulfonyl-benzoesäure werden in 100 ml $SOCl_2$ nach Zu-

gabe von 0,5 ml DMF 2 Stunden unter Rückfluß gekocht. Nach Destillation des überschüssigen Thionylchlorids im Vakuum und Digerieren des Rückstandes mit Äther wird das Säurechlorid in 100 ml $CHCl_3$ gelöst und bei 0 - 10° zu einer Lösung von 15,1 g (0,11 Mol) 2-Amino-1-phenyl-äthanol-1 und 16,7 ml (0,12 Mol) Triäthylamin in 100 ml $CHCl_3$ zugetropft. Man läßt auf RT kommen, rührt 3 Stunden nach, destilliert das Chloroform und zieht den Rückstand mit 400 ml 0,5 N HCl aus, wäscht mit Wasser nach, trocknet und kristallisiert aus Methanol.
Kristalle vom Schmp. 169 - 170°

Beispiel 2
Cyclisierung der Hydroxyalkylamide der Formel II

a) mittels Thionylchlorid

20 g (0,07 Mol) 4-Chlor-3-sulfamoylbenzoesäure (2-hydroxy-äthyl- )-amid werden eine halbe Stunde in 80 ml Thionylchlorid, dem einige Tropfen DMF zugefügt sind, unter Rückfluß gekocht, danach wird abgekühlt und im Vakuum das überschüssige Thionylchlorid destillativ entfernt. Nach Anreiben des Rückstandes mit Äther wird der Kristallbrei abgesaugt, mehrfach mit Äther gewaschen, kurz getrocknet und bei 0° in 40 ml 33 %ige NaOH eingetragen. Nach einstündigem Rühren wird mit 500 ml kaltem Wasser versetzt und das 4-Chlor-3-sulfamoyl- (oxazolin-2-yl)-benzol mit 4 n HCl in der Kälte ( 0 - 10°) bei pH 9 gefällt. Der Niederschlag wird abgesaugt, mehrfach mit Wasser gewaschen, getrocknet und aus Dioxan umkristallisiert. Kristalle vom Schmp. 186 - 187°

b) Analog a) erhält man aus 4-Chlor-3-sulfamoylbenzoesäure-(1-hydroxy-2-methyl-propyl-2)-amid das 4-Chlor-3-sulfamoyl-(4,4-dimethyl-oxazolin-2-yl)-benzol vom Schmp. 218 - 220° (aus Methanol).

c) Analog a) erhält man aus 4-Chlor-3-sulfamoylbenzoesäure-(2-hydroxy-propyl-3)-amid das 4-Chlor-3-sulfamoyl-(5-methyloxazolin-2-yl)-benzol.
Kristalle vom Schmp. 182 - 184° aus Methanol.

d) 4-Chlor-3-sulfamoyl- (4-äthyloxazolin-2-yl)-benzol

Zu einer Lösung von 48,6 g (~0,16 Mol) 4-Chlor-3-sulfamoylbenzoesäure(1-hydroxy-butyl-2)-amid in 98 ml $SOCl_2$ werden bei 10° 0,3 ml DMF gegeben. Es wird bei Raumtemperatur 30 Minuten nachgerührt. Sobald eine klare Lösung entstanden ist, wird bei Raumtemperatur das überschüssige $SOCl_2$ im Vakuum abdestilliert und der Rückstand mehrfach mit Äther digeriert. Der Rückstand wird kurz getrocknet und langsam in 100 ml kalte 35 %ige NaOH eingetragen. Nach halbstündigem Rühren bei Raumtemperatur wird mit 4 n HCl bei pH 9 gefällt. Aus Nitromethan wird umkristallisiert.
Kristalle vom Schmp. 129 - 130°

e) 4-Brom-3-sulfamoyl- (4-äthyl-oxazolin-2-yl)-benzol

erhält man analog d) aus 4-Brom-5-sulfamoyl(1-hydroxy-butyl-2)-amid.
Kristalle (aus Methanol) vom Schmp. 165 - 166°

f) 4-Chlor-3-sulfamoyl- (5-phenyl-oxazolin-2-yl)-benzol

1.8 g (0,0225 Mol) 4-Chlor-5-dimethylaminomethylen-aminosulfonyl-benzoesäure-(2-hydroxy-2-phenyl-äthyl)-amid werden in 32 ml Thionylchlorid nach Zugabe einiger Tropfen DMF 1/2 Stunde unter Rückfluß gekocht. Das überschüssige Thionylchlorid wird bei Raumtemperatur im Vakuum abdestilliert und der Rückstand mit Äther gefällt. Nach Eintragen des Rückstandes in 18 ml 33 %ige NaOH wird mit 50 ml Methanol verdünnt und 45 Minuten bei 60° gerührt, danach wird mit 300 ml Wasser

verdünnt und in der Kälte bei 0 - 10° das Oxazolinderivat bei pH 9 gefällt. Der Niederschlag wird abgesaugt, mehrfach mit Wasser gewaschen, getrocknet und aus Methanol umkristallisiert.
Kristalle vom Schmp. 196 - 197°

Beispiel 3

Ringschluß über die Bildung eines Tosylats

4-Chlor-3-sulfamoyl-(4,4 -dimethyl-oxazolin-2-yl)-benzol

7,7 g (0,025 Mol) 4-Chlor-3-sulfamoylbenzoesäure-(1-hydroxy-2-methyl-propyl-2-)amid werden in 12 ml Pyridin gelöst und auf 0° gekühlt. Zu dieser Lösung werden portionsweise 4,8 g (0,025 Mol) Tosylchlorid zugegeben. Nach zweistündigem Rühren bei Raumtemperatur wird in 80 ml eiskalte 10 %ige HCl eingetragen und in der Kälte bei 0 - 10° das Oxazolinderivate mit 2 n NaOH bei pH 9 gefällt. Der Niederschlag wird abgesaugt, mehrfach mit Wasser gewaschen, getrocknet und aus Methanol umkristallisiert.
Schmp. 218 - 220°

Beispiel 4

Ringschluß durch Erhitzen über den Schmelzpunkt

4-Chlor-3-sulfamoyl-(4,4-dimethyl-oxazolin-2-yl)-benzol

24 g ( 0,1 Mol) 4-Chlor-3-sulfamoylbenzoesäure-(1-hydroxy-2-methyl-propyl-2)-amid werden 1 Std. über den Schmelzpunkt auf 190 - 200° erhitzt. Nach beendeter Bläschenbildung wird auf 0° gekühlt. Der Rückstand wird in 2 n NaOH gelöst, die Lösung filtriert und das Oxazolinderivat bei 0 - 10° mit 2 n HCl bei pH 9 gefällt. Der Niederschlag wird abgesaugt mit Wasser gewaschen und zweimal aus Methanol umkristallisiert.
Kristalle vom Schmp. 218 - 220°

Beispiel 5 (Variante b)

4-Chlor-3-sulfamoyl-4-(5-methyl-oxazolin-2-yl)-benzol

10 g (0,03 Mol) 4-Chlor-3-sulfamoylbenziminoäthylester-hydrochlorid werden mit 5,1 ml (0,06 Mol) 1-Amino-propanol-2 verrieben. Nach einstündigem Rühren werden 30 ml 2 n NaOH zugefügt, mit Wasser verdünnt und die Lösung mit 2 n HCl auf pH 9 angesäuert. Nach Stehen über Nacht bei 0° wird der Niederschlag des Oxazolinderivats abgesaugt, mit kaltem Wasser gewaschen, getrocknet und aus Methanol umkristallisiert.
Kristalle vom Schmp. 182 - 184°

Herstellung des Ausgangsmaterials 4-Chlor-3-sulfamoyl-benziminoäthylester-hydrochlorid:

In eine Suspension von 20 g 4-Chlor-3-sulfamoyl-benzonitril in 100 ml absolutem Äthanol wird bei 0° 3 Std. HCl-Gas eingeleitet. Nach dreistündigem Stehen bei 0° wird der feste Rückstand mit Äther versetzt. Das Hydrochlorid des Benziminoäthyläther-Derivats wird abgesaugt, sorgfältig mit Äther gewaschen und getrocknet.
Kristalle vom Schmp. 151 - 153°

Beispiel 6 (Methode d)

4-Chlor-3-sulfamoyl- (oxazolin-2-yl)-benzol

5 g (0,023 Mol) 4-Chlor-3-sulfamoylbenzonitril werden bei -5° in 10 ml konzentrierte $H_2SO_4$ eingetragen. Unter Rühren tropfen innerhalb 1 Stunde 2,3 ml (0,046 Mol) Äthylenoxid zu. Nach vierstündigem Nachrühren bei +5° wird auf Eiswasser gegeben, nicht umgesetztes 4-Chlor-3-sulfamoylbenzonitril abgesaugt und das Filtrat mit 4 n NaOH auf pH 9 eingestellt. Nach Stehen über Nacht im Eisschrank wird das ausgefallene Oxazolinderivat abgesaugt, getrocknet und aus Dioxan umkristallisiert.
Kristalle vom Schmp. 186 - 187°

Beispiel 7 (Methode e)

4-Chlor-3-sulfamoyl- (5-methyl -oxazolin-2-yl)-benzol

Eine Lösung von 2,75 g (∼0,1 Mol) 4-Chlor-3-sulfamoyl-benzoesäure-allylamid in 5 ml konzentrierter $H_2SO_4$ werden 3 Std. auf 90° erhitzt. Die Lösung wird abgekühlt, auf Eis gegeben, die kalte Lösung mit Kohle geklärt und das Oxazolinderivat bei 10° mit 2 N NaOH bei pH 9 gefällt. Der Niederschlag wird abgesaugt, mehrfach mit Wasser gewaschen, getrocknet und aus Methanol umkristallisiert.
Kristalle vom Schmp. 182 - 184°

Beispiel 8

4-Chlor-3-sulfamoyl-(4,4-dimethyl-oxazolin-2-yl)-benzol hydrochlorid

2,9 g (∼ 0,01 Mol) 4-Chlor-3-sulfamoyl-(4,4-dimethyl-oxazolin-2-yl)-benzol werden in 100 ml Aceton suspendiert. Nach Zugabe der berechneten Menge (5 ml ∼ 0,011 Mol) 2 n HCl löst sich die Suspension. Kurze Zeit später fällt beim gelinden Erwärmen das HCl-Salz des Oxazolinderivats aus. Der Niederschlag wird abgesaugt, gründlich mit Aceton gewaschen und im Hochvakuum getrocknet.
Kristalle vom Schmp. 231 - 232°

Beispiel 9

Umlagerung der Verbindungen der Formel I b

a) 4-Chlor-3-sulfamoylbenzoesäure-(2-amino-äthyl)-ester-hydrochlorid

2,6 g (0,01 Mol) 4-Chlor-3-sulfamoyl- (oxazolin-2-yl)-benzol werden unter Rühren in 10 ml 2 n HCl ca. 2 Minuten auf 80 - 90° erhitzt. Sobald eine klare Lösung entstanden ist, wird abgekühlt und der Ester mit einem Überschuß Aceton gefällt. Die Niederschlag wird abgesaugt und im Hochvakuum getrocknet.
Kristalle vom Schmp. 233 - 234°

b) 4-Chlor-3-sulfamoylbenzoesäure-(2-methyl-2 -amino-propyl)-ester-hydrochlorid

In analoger Weise wird das 4-Chlor-3-sulfamoylbenzoesäure-(2-methyl-2 -amino-propyl)-ester-hydrochlorid vom Schmp. 264° erhalten.

Beispiel 10

4-Chlor-3-sulfamoyl- (oxazolin-2-yl)-benzol

Zu einer Lösung von 23,5 g (0,1 Mol) 4-Chlor-3-sulfamoylbenzamid in 77 ml (1 Mol) DMF werden bei -10° 36 ml (0,5 Mol) Thionylchlorid zugetropft. Man läßt auf Raumtemperatur kommen, rührt 3 Stunden nach und gibt auf Eiswasser. Der Niederschlag wird abgesaugt und aus DMF/Methanol umkristallisiert. Man erhält 4-Chlor-3-dimethylaminomethylenaminosulfonyl-benzonitril vom Schmp. 169°.

Die Umsetzung mit Äthylenoxyd erfolgt wie in Beispiel 6 beschrieben. Im Anschluß an diese Umsetzung wird durch alkalische Hydrolyse die Schutzgruppe abgespalten und das Endprodukt vom Schmp. 186° erhalten.

**Beispiel 11**

**4-Chlor-3-sulfamoyl-(4-methyl-5-phenyl-oxazolin-2-yl)-benzol**

Zu einer Lösung von 12 g (0,04 Mol) 4-Chlor-3-sulfamoyl-iminoäthylester-hydrochlorid, 7,5 g (0,04 Mol) Norephedrin-hydrochlorid in 70 ml absolutem Äthanol werden 11,2 ml (0,08 Mol) Triäthylamin zugetropft. Nach einstündigem Rühren bei 60$^{o}$ wird abgekühlt in Eiswasser eingetragen. Der Niederschlag wird aus Methanol umkristallisiert.

Kristalle vom Schmp.: 206 - 208 $^{o}$C

Patentansprüche:

1. Halogensulfamoylbenzole der allgemeinen Formel

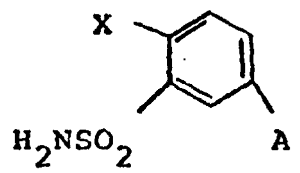

I a bzw. I b

in der X für ein Halogen und A für einen der Reste

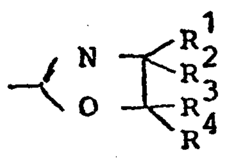

oder

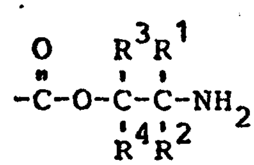

a b

steht, wobei die Reste $R^1$ bis $R^4$ gleich oder verschieden sind und Wasserstoff oder einen Alkylrest mit 1 - 4 C-Atomen bedeuten, einer der Substituenten $R^1$ bis $R^4$ eine Carboxy-, Hydroxymethyl- oder eine Alkyloxycarbonyl-Gruppe mit höchstens 5 C-Atomen bedeuten oder ein oder zwei der Substituenten $R^1$ bis $R^4$ iso-Propyl, iso-Butyl, tert.-Butyl, Phenyl oder Cycloalkyl mit 5 - 6 C-Atomen bedeuten, während die übrigen für Wasserstoff oder niederes Alkyl stehen, sowie deren Salze mit physiologisch verträglichen Säuren.

2. Verfahren zur Herstellung der Verbindungen der Formel I, dadurch gekennzeichnet, daß man

a) Sulfamoylverbindungen der Formel II

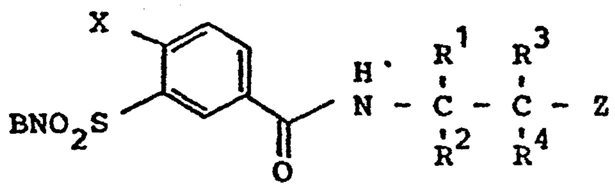

II

in der die Reste $R^1$ bis $R^4$ und X die angegebene Bedeutung haben und B für 2 Wasserstoffatome oder eine Schutzgruppe der Formel

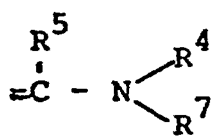

steht, in der die Reste $R^5$ bis $R^7$ gleiche oder verschiedene Alkylgruppen mit 1 - 4 C-Atomen bedeuten, wobei $R^5$ auch für Wasserstoff stehen kann und Z eine leaving group darstellt, cyclisiert oder

b) Sulfamoylverbindungen der Formel III

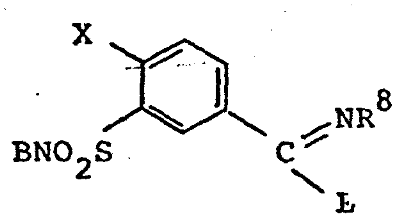

III

in der X und B die angegebenen Bedeutungen besitzen, $R^8$ Wasserstoff oder einen Alkylrest mit 1 - 4 C-Atomen bedeutet und L für Halogen, Alkoxy oder Dialkylamino, steht, worin die Alkyl- bzw. Alkoxyreste 1 - 4 C-Atome enthalten, in Form ihrer Salze mit Aminoalkoholen der allgemeinen Formel IV

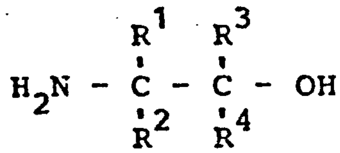

IV

in der die Reste $R^1$ bis $R^4$ die angegebene Bedeutung besitzen, umsetzt oder

c) Sulfamoylverbindungen der allgemeinen Formel V

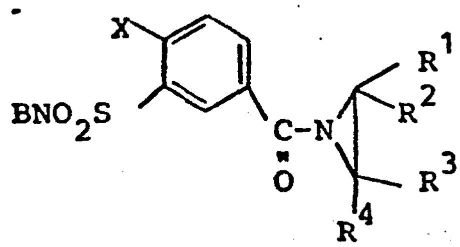

V

in der die Reste $R^1$ bis $R^4$, X und B die angegebene Bedeutung haben, durch Erwärmen oder Zusatz eines Katalysators zum Oxazolinderivat der allgemeinen Formel I a isomerisiert oder

d) ein Nitril der Formel VI

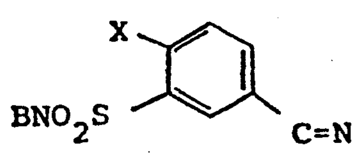

VI

in der X und B die angegebene Bedeutung besitzen, mit Epoxiden der allgemeinen Formel VII

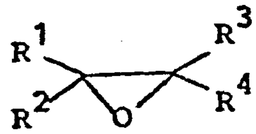

VII .

worin $R^1$ bis $R^4$ die angegebene Bedeutung besitzen, jedoch nicht für Phenyl stehen, in Gegenwart starker Säuren umsetzt oder

e) ein Allylamid der Formel VIII

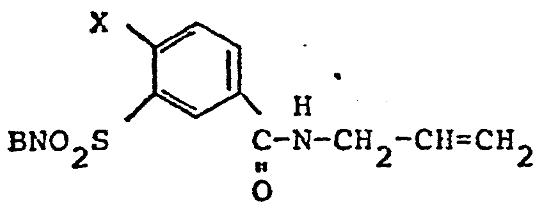

VIII

in der X und B die angegebene Bedeutung haben, mit Säuren cyclisiert,

und in den nach a) bis e) erhaltenen Verbindungen eine gegebenenfalls vorhandene Schutzgruppe durch alkalische Hydrolyse abspaltet und gegebenenfalls die nach a) bis e) erhaltenen Oxazolinderivate der Formel I a in Gegenwart von wäßrigen Säuren in der Wärme zu Verbindungen der Formel I b umlagert und/oder die Verbindungen der Formel I a bzw. I b mit physiologisch verträglichen organischen oder anorganischen Säuren in die Säure-Additionssalze und/oder die anfallenden Salze in die entsprechenden Basen überführt.

3. Pharmazeutische Zubereitungen mit salidiuretischer Wirkung, gekennzeichnet durch einen Gehalt an Verbindungen gemäß Anspruch 1 und gegebenenfalls von üblichen pharmazeutischen Trägerstoffen und/oder Stabilisatoren.

4. Verfahren zur Herstellung von pharmazeutischen Zubereitungen gemäß Anspruch 3, dadurch gekennzeichnet, daß man eine Verbindung gemäß Anspruch 1 ggf. unter Zusatz von pharmazeutischen Trägerstoffen und/oder Stabilisatoren in eine für pharmazeutische Zwecke geeignete Anwendungsform bringt.

**Patentanspruch für Österreich:**

Verfahren zur Herstellung von Halogensulfamoylbenzolen der allgemeinen Formel

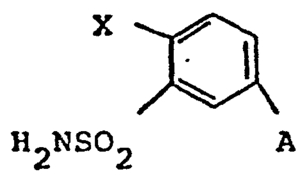

I a bzw. I b

in der X für ein Halogen und A für einen der Reste

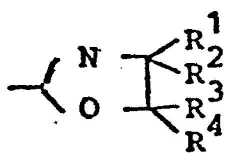

oder

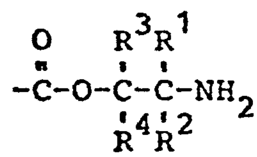

a b

steht, wobei die Reste $R^1$ bis $R^4$ gleich oder verschieden sind und Wasserstoff oder einen Alkylrest mit 1 - 4 C-Atomen bedeuten, einer der Substituenten $R^1$ bis $R^4$ eine Carboxy-, Hydroxymethyl- oder eine Alkyloxycarbonyl-Gruppe mit höchstens 5 C-Atomen bedeuten oder ein oder zwei der Substituenten $R^1$ bis $R^4$ iso-Propyl, iso-Butyl, tert.-Butyl, Phenyl oder Cycloalkyl mit 5 - 6 C-Atomen bedeuten, während die übrigen für Wasserstoff oder niederes Alkyl stehen, sowie von deren Salzen mit physiologisch verträglichen Säuren, dadurch gekennzeichnet, daß man

a) Sulfamoylverbindungen der Formel II

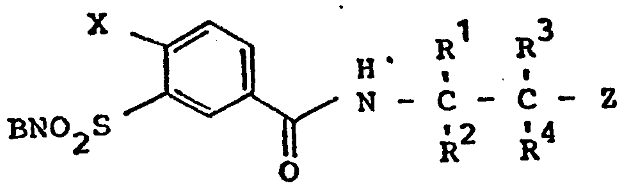

II

in der die Reste $R^1$ bis $R^4$ und X die angegebene Bedeutung haben und B für 2 Wasserstoffatome oder eine Schutzgruppe der Formel

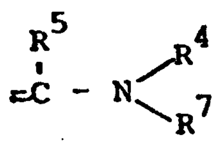

steht, in der die Reste $R^5$ bis $R^7$ gleiche oder verschiedene Alkylgruppen mit 1 - 4 C-Atomen bedeuten, wobei $R^5$ auch für Wasserstoff stehen kann und Z eine leaving group darstellt, cyclisiert oder

b) Sulfamoylverbindungen der Formel III

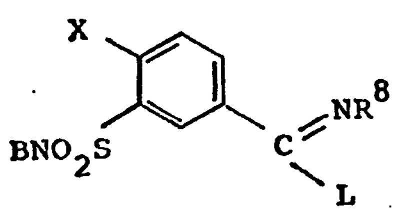

III

in der X und B die angegebenen Bedeutungen besitzen, $R^8$ Wasserstoff oder einen Alkylrest mit 1 - 4 C-Atomen bedeutet und L für Halogen, Alkoxy oder Dialkylamino steht, worin die Alkyl- bzw. Alkoxyreste 1 - 4 C-Atome enthalten, in Form ihrer Salze mit Aminoalkoholen der allgemeinen Formel IV

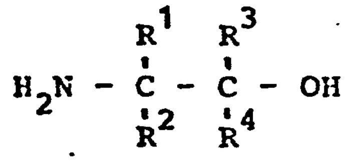

IV

in der die Reste $R^1$ bis $R^4$ die angegebene Bedeutung besitzen, umsetzt oder

c) Sulfamoylverbindungen der allgemeinen Formel V

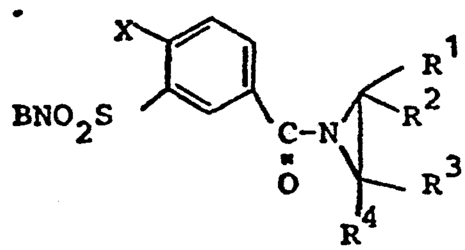

V

in der die Reste $R^1$ bis $R^4$, X und B die angegebene Bedeutung haben, durch Erwärmen oder Zusatz eines Katalysators zum Oxazolinderivat der allgemeinen Formel I a isomerisiert oder

d) ein Nitril der Formel VI

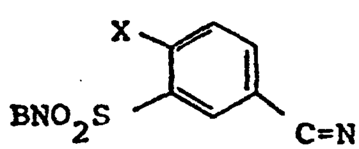

VI

in der X und B die angegebene Bedeutung besitzen, mit Epoxiden der allgemeinen Formel VII

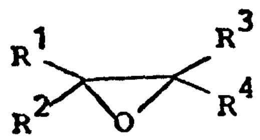

VII .

worin $R^1$ bis $R^4$ die angegebene Bedeutung besitzen, jedoch nicht für Phenyl stehen, in Gegenwart starker Säuren umsetzt oder

e) ein Allylamid der Formel VIII

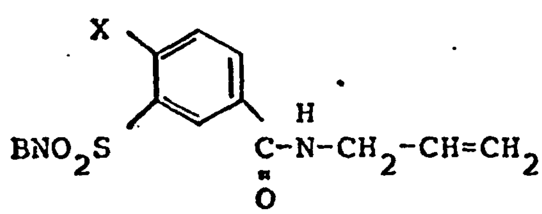

VIII

in der X und B die angegebene Bedeutung haben, mit Säuren cyclisiert,

und in den nach a) bis e) erhaltenen Verbindungen eine gegebenenfalls vorhandene Schutzgruppe durch alkalische Hydrolyse abspaltet und gegebenenfalls die nach a) bis e) erhaltenen Oxazolinderivate der Formel I a in Gegenwart von wäßrigen Säuren in der Wärme zu Verbindungen der Formel I b umlagert und/oder die Verbindungen der Formel I a bzw. I b mit physiologisch verträglichen organischen oder anorganischen Säuren in die Säure-Additionssalze und/oder die anfallenden Salze in die entsprechenden Basen überführt.

0008433

Europäisches Patentamt

# EUROPÄISCHER RECHERCHENBERICHT

Nummer der Anmeldung
EP 79 10 2951

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | GB - A - 915 259 (PARKE, DAVIS & CY)<br>* Vollständig *<br>-- | 1-4 |
| | FR - A - 1 311 859 (SIFA)<br>* Vollständig *<br>-- | 1-4 |
| | US - A - 3 452 037 (A.A. SANTILLI)<br>* Vollständig *<br>---- | 1-4 |

KLASSIFIKATION DER ANMELDUNG (Int.Cl. 3)

C 07 D 263/10
C 07 C 143/78
A 61 K 31/42
A 61 K 31/235

RECHERCHIERTE SACHGEBIETE (Int. Cl. 3)

C 07 D 263/10
C 07 C 143/78
A 61 K 31/42
A 61 K 31/235

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 15-11-1979 | ALLARD |